# FASCICULE DE BREVET EUROPEEN

(11) **EP 0 424 444 B1**
(45) Date de publication et mention de la délivrance du brevet: **14.09.1994**
(21) Numéro de dépôt: 89908204.4
(22) Date de dépôt: 12.07.1989
(51) Int. Cl.: A61K 7/48

(54) **COMPOSITIONS COSMETIQUES A BASE DE CHROMONE-CARBOXYLATE**
KOSMETISCHE ZUSAMMENSETZUNG AUF DER BASIS VON CHROMO-CARBOXYLATEN
COSMETIC COMPOSITIONS BASED ON CHROMONE-CARBOXYLATE

(30) Priorité: 13.07.1988 FR 8809525
(43) Date de publication de la demande: 02.05.1991
(73) Titulaire: TRANSPHYTO S.A., 63016 Clermont Ferrand (FR)
(72) Inventeur: BONIFACE, Robert, F-95810 Vallangoujard (FR)
(74) Mandataire: Thibon-Littaye, Annick
(86) Numéro de dépôt international: FR8900372
(87) Numéro de publication internationale: WO9000388

(56) Documents cités:
- EP-A- 0 238 302
- EP-A- 0 304 802
- GB-A- 1 185 796
- GB-A- 1 475 503
- Chemical Abstracts, vol. 94, 1981 (Columbus, Ohio, US) see p. 361, abstract
- no. 145182m
- Chemical Abstracts, vol. 102, 1985 (Columbus, Ohio, US) J. Swarbrick et al:"Drug permeation through human skin. II: Permeability of ionizable compounds"see p. 11, abstract no. 17069b

## Description

La présente invention concerne principalement l'utilisation de composés chimiques en eux-mêmes connus, dans la fabrication de nouveaux cosmétiques, qui se sont révélés efficaces, en application locale sur la peau, pour lutter contre le vieillissement de celle-ci. Elle a donc également pour objet les préparations cosmétiques, destinées à être appliquées localement sur la peau, qui contiennent un ou plusieurs desdits composés comme constituant actif contre le vieillissement de la peau.

Les composés chimiques dont il est fait usage sont les sels d'acide chromone-carboxylique, et plus particulièrement les chromone-carboxylates-2 d'amines basiques ou de métaux alcalins ou alcalino-terreux. Ils ont été décrits dans leur préparation et dans leurs propriétés chimiques dans le brevet spécial de médicament FR-6 932 M, qui proposait d'utiliser dans le domaine pharmaceutique les sels organiques de l'acide chromone-carboxylique-2, en raison de leur activité vitaminique P. Depuis lors, le chromone-carboxylate-2 de diéthylamine s'est effectivement révélé efficace dans une activité générale sur l'organisme humain, quand il est administré par voie orale ou parentérale pour augmenter la résistance capillaire et diminuer la perméabilité capillaire, et traiter ainsi l'insuffisance circulatoire et les maladies vasculaires.

Le même brevet proposait en outre d'incorporer ce composé dans des collyres. Il s'agissait alors d'une thérapeutique vasculaire locale, directement sur la muqueuse de l'oeil. Rien pour autant n'aurait permis de prévoir qu'il présenterait d'autres activités en application sur la peau, et non plus sur une muqueuse. C'est ce que révèle la présente invention. Conformément à celle-ci, on a en effet découvert que ce composé et les composés analogues sont efficaces contre le vieillissement de la peau quand ils sont appliqués sur l'épiderme cutané, en tant que produits cosmétiques tels que les crèmes, laits, gels et lotions dermiques.

L'invention a donc notamment pour objet des compositions cosmétiques pour application locale sur l'épiderme cutané, ainsi qu'un procédé de traitement cosmétique de la peau, suivant lequel on applique sur l'épiderme cutané une composition comportant comme constituant actif contre le vieilissement de la peau, un sel d'acide chromone-carboxylique, en mélange avec un excipient classique.

Un tel excipient peut être notamment celui d'une lotion ou d'un gel hydrique ou hydroalcoolique, ou celui d'une crème ou d'un lait, auquel cas il peut avantageusement comporter, en pourcentage en poids du poids total de la composition, celle-ci étant sous la forme d'une émulsion huile dans eau :

| | |
|---|---|
| Conservateurs | 0,1 à 0,5 |
| Emulsionnants non ioniques | 10 à 25 |
| Huiles végétales et synthétiques | 5 à 15 |
| Facteurs hydratants | 1 à 10 |

Le sel actif incorporé dans les compositions selon l'invention y est de préférence présent dans une proportion pondérale comprise entre 1 et 10 % du poids total de la composition, et notamment entre 2 et 8 %. Ce peut être soit un sel organique d'acide chromone-carboxylique tel que, de préférence le chromone-carboxylate-2 de diéthylamine, soit un sel minéral, alcalin ou alcalino-terreux d'acide chromone-carboxylique, tel que de préférence les chromone-carboxylate-2 de sodium, potassium, ou magnésium.

Sous cette forme nouvelle de compositions cosmétiques, et en application locale sur l'épiderme cutané, ces sels d'acide chromone-carboxylique ont fait preuve d'une efficacité dans la lutte contre le vieillissement de la peau qui n'était ni envisagée ni prévisible antérieurement à la présente invention, y compris au vu des expériences faites dans le cadre de l'activité pharmaceutique connue sur la résistance et la perméabilité capillaires. Ils remplacent donc avec profit les substances naturelles d'origine végétale ou animale qui sont utilisées habituellement pour ce genre d'application.

On a pu constater en effet qu'ils agissent de façon significative et inédite sur les principaux paramètres reconnus du vieillissement cutané naturel ou accéléré quand ces compositions sont appliquées régulièrement sur la peau de sujets vivants. En particulier, leur utilisation entraîne ou permet :
. une évolution de la répartition quantitative des fibres de collagène "soluble" et "insoluble" dans le derme (valeur du rapport collagène soluble/collagène insoluble), allant dans le sens de celle que l'on observe au cours de la jeunesse ;
. une augmentation du stockage des lipides, qui permettent une lubrification constante du revêtement cutané, alors que ce facteur est reconnu comme déficitaire au cours du vieillissement, avec ses conséquences sur la souples se de la peau, sa sécheresse et son aspect rêche au toucher ;
. une réduction de la dégradation des structures fibreuses de la peau par les enzymes protéolytiques, dont l'action est bien connue et démontrée comme facteur de vieillissement et dont la libération par les cellules épithéliales est augmentée par l'exposition de l'épithélium aux diverses agressions exogènes dont il est l'objet dans la vie courante, tels que les facteurs météorologiques, les agressions photoniques, la pollution atmosphérique, le tabagisme, le rasage ;
. une protection contre les rayonnements solaires nocifs pour la peau.

L'invention sera maintenant explicitée et illustrée au moyen d'exemples de compositions cosmétiques et de résultats expérimentaux. Sauf indication contraire, les quantités et proportions sont exprimées en poids.

### EXEMPLES DE COMPOSITIONS COSMETIQUES.

Dans les exemples donnés ci-après, les constituants de l'excipient sont souvent désignés par leurs dénominations commerciales. Ce sont notamment :
- des conservateurs : Kathon CG (chlorom ethyl-isothiazolone), Nipagine (Paraben, ester méthylique), Nipasol (Paraben, ester éthylique), acide sorbique ;
- des émulsionnants, choisis pour leur miscibilité à des solvants divers et leur capacité de maintenir le sel actif dans les zones d'application en vue de sa libération de l'excipient : stéarate de polyéthylène-glycol, Téfose (palmito-stéarate de polyglycols), Labrafil (glycérides de polyoxyéthylène glycolysés) ;
- des facteurs hydratants : propylène-glycol pour 4 à 8 % de la composition totale, allantoïne pour 1 à 2 %, urée ou mélange d'urée et d'acide lactique pour 5 à 10 %, facteur NMF ou Natural Moisturizing Factor pour 3 à 5 % ;
- des huiles végétales ou synthétiques : huile d'onagre, Cétiol B (dibutyl-adipate), huile de silicone, huile de paraffine fluide, éventuellement en mélange (pour 8 à 12 % ;
- des agents gélifiants comme : CARBOPOL 940 (carboxypolyméthylène), Métolose (hydroxypropylméthylcellulose), pour à 0,2 à 2 % de la composition totale ;
- des additifs divers tels que : Emulgin (alcool cétylstéarylique), Aloe Vera Gel comme agent cicatrisant, Enoxolone (acide 18-bêta-glycyrrhétinique) comme agent anti-inflammatoire.

### Exemple I : Crème de jour

Pour la protection de la peau contre les agressions extérieures, on applique chaque matin la valeur approximative d'une noisette de la formule ci-après :

| | Grammes |
|---|---|
| Chromone-carboxylate de diéthylamine | 5 |
| Téfose 1500 | 15 |
| Labrafil M 2130 CS | 2 |
| Acide stéarique | 2 |
| Cétiol B | 8 |
| Huile de silicone fluide | 2 |
| Propylène glycol | 7 |
| Allantoïne | 1,5 |
| Kathon CG | 0,25 |
| Parfum q.s. | |
| Eau désionisée q.s.p. | 100 |

### Exemple II : Crème de nuit

Pour le traitement de nuit, on applique chaque soir la formule ci-après :

| | Grammes |
|---|---|
| Chromone-carboxylate de diéthylamine | 5 |
| Téfose 1500 | 18 |
| Labrafil M 2130 CS | 2 |
| Acide stéarique | 2 |
| Huile d'onagre | 2 |
| Cétiol B | 10 |
| Glycérine | 7 |
| Facteur NMF | 4 |
| Kathon CG | 0,25 |
| Parfum | q.s. |
| Eau désionisée | q.s.p. 100 |

### Exemple III : Crème de nuit

En variante de l'exemple précédent, on utilise une crème de nuit qui contient, dans le même excipient, seulement 2,5 g de chromone-carboxylate-2 de diéthylamine (CCD).

### Exemple IV :

En variante des exemples précédents, on utilise des crèmes dermiques où, dans les mêmes excipients, le chromone-carboxylate-2 de diéthylamine est remplacé par l'un des composés suivants, dans des quantités comprises entre 3 et 6 g pour 100 g de crème :

| | |
|---|---|
| Chromone-carboxylate-2 de sodium | 5 |
| Chromone-carboxylate-2 de potassium | 4 |
| Chromone-carboxylate-2 de magnésium | 6 |

### Exemple V : Gel solaire

Pour la protection solaire préventive, on applique plusieurs fois par jour, en particulier après chaque bain, la formule suivante :

| | Grammes |
|---|---|
| Chromone-carboxylate de diéthylamine | 5 |
| Carbopol 940 | 0,3 |
| Métolose 65 SH 4000 | 1 |
| Nipagine | 0,3 |
| Triéthanolamine | q.s. |
| Parfum | q.s. |
| Eau désionisée | q.s.p. 100 |

### Exemple VI : Lait apaisant après soleil

Pour limiter les effets nocifs du soleil sur la peau, on applique plusieurs fois après l'exposition au soleil, et pendant plusieurs jours consécutifs si nécessaire, l'émulsion de type huile dans eau suivante :

| | Grammes |
|---|---|
| Chromone-carboxylate de diéthylamine | 3 à 5 |
| Téfose 1500 | 5 |
| Emulgin B1 | 0,3 |
| Emulgin B2 | 0,7 |
| Cétiol B | 4 |
| Huile de silicone | 1 |
| Acide stéarique | 1,5 |
| Aloe Vera Gel | 5 |
| Propylène glycol | 5 |
| Nipagine | 0,2 |
| Nipasol | 0,1 |
| Parfum q.s. | |
| Eau désionisée | q.s.p.100 |

En variante, le même lait est préparé en remplaçant le chromone-carboxylate de diéthylamine par 3,3 g de chromone-carboxylate de magnésium.

### Exemple VII : Gel apaisant

Dans les mêmes conditions que dans l'exemple précédent, on utilise un gel hydroalcoolique présentant la formule suivante :

| | Grammes |
|---|---|
| Chromone-carboxylate de diéthylamine | 3 |
| Enoxolone | 0,5 |
| Allantoïne | 1 |
| Carbopol 940 | 0,4 |
| Propylène glycol | 7 |
| Nipagine | 0,3 |
| Triéthanolamine | 0,8 |
| Ethanol à 95 % | 20 |
| Eau désionisée | q.s.p.100 |

### Exemple VIII : Lotion apaisante

Pour calmer le feu du rasoir ou pour calmer tout autre type d'irritation, on utilise une lotion présentant la formule ci-après :

| | Grammes |
|---|---|
| Chromone-carboxylate de diéthylamine | 3 |
| Menthol | 0,05 |
| Enoxolone | 0,5 |
| Allantoïne | 1 |
| Propylène glycol | 7 |
| Nipagine | 0,3 |
| Ethanol à 95 % | 20 |
| Parfum | q.s. |
| Eau désionisée | q.s.p. 100 |

### ESSAIS D'EFFICACITE COMPARATIFS

### Exemple IX : Spectre d'absorption UV :

Le spectre d'absorption du chromone-carboxylate-2 de diéthylamine (CCD) a été réalisé dans l'eau à la concentration de 0,02 g dans 100 litres d'eau. On observe des maxima à 312 nm (UVB), 276 et 236 nm (UVC) et 206 nm.

On constate donc que le spectre répond aux critères de sélection d'un filtre solaire. Le coefficient d'extinction moléculaire est supérieur à 7000.

Cette filtration est un des facteurs de protection cutanée apportée par le CCD contre les agents exogènes d'altération épidermique.

### Exemple X : Action sur la teneur en lipides de la peau

Les expérimentations rapportées ici ont été faites sur deux séries de rats Wister mâles de 400 à 420 g provenant du même élevage, qui ont été traités par la même personne, afin d'obtenir des échantillons de la meilleure homogénéité.

Une première série de 30 animaux étaient traités avec une crème dermique selon l'invention, et une autre série de 30 animaux avec une crème classique, constituée du mélange utilisé comme excipient dans la crème de l'invention. Sur chaque animal, la crème était appliquée une fois par jour, quotidiennement, sur un flanc préalablement tondu, alors que sur l'autre flanc tondu on appliquait du sérum physiologique à titre de témoin.

Après 40 applications en 6 semaines, les peaux traitées ont été prélevées sur les animaux sacrifiés et découpées en petits carrés pour être examinées par détermination de la teneur en eau et dosage des lipides et des protéines.

La détermination de la teneur en eau porte sur des échantillons de 5 g de chaque peau, dont le poids frais est comparé au poids sec après dessiccation par lyophilisation, la différence de poids représentant la teneur en eau.

Pour déterminer la teneur en lipides libres, des échantillons de peau de 2 g sont finement broyés et mélangés au réactif de Folch (chloroforme/éthanol : 3/1 en volume). Après agitation pendant une heure, puis filtration, le résidu est repris au même réactif puis filtré, l'opération étant répétée quatre fois. Les extraits obtenus sont réunis pour être soumis à évaporation jusqu'à obtention d'un poids constant qui est celui des lipides libres.

La teneur en lipides liés est également déterminée par gravimétrie. Ceci implique la destruction de la trame collagénique par extraction des résidus au tampon calcique neutre puis à l'acide trichloracétique. Le résidu de l'extraction est traité par le réactif de Folch, puis par évaporation à trois reprises dans les mêmes conditions que pour les lipides libres. Par double pesée on obtient le poids des lipides liés.

En examinant ainsi les différentes compositions indiquées dans les exemples précédents, par comparaison avec leurs excipients respectifs, on constate à chaque fois une augmentation sensible du taux d'hydratation de la peau et de la rétention des lipides.

Les dosages ainsi effectués sur l'ensemble des échantillons de peau prélevés sur 30 animaux conduisent aux taux moyens suivants dans le cas de la crème de l'exemple 1 :

| Lipides | totaux mg/g | libres mg/g | liés mg/g |
|---|---|---|---|
| Excipient | | | |
| Témoin | 211,80 | 99,32 | 112,49 |
| Excipient | 234,75 | 117,00 | 117,74 |

| Crème de l'invention | | | |
|---|---|---|---|
| Témoin | 213,29 | 104,61 | 108,67 |
| Crème | 227,77 | 105,07 | 122,69 |

Il apparaît ainsi que là où la crème classique (excipient) entraîne une augmentation des lipides totaux qui est surtout due aux lipides libres, les lipides liés restant très stables, l'augmentation constatée avec la crème selon l'invention se manifeste surtout sur les lipides liés (augmentation de 16 %), ce qui traduit une fixation avantageuse des lipides sur le stroma.

Physiologiquement, cette fixation sur le stroma correspond à un stockage qui assure une constante imprégnation lipidique de la peau, lui conservant ainsi sa souplesse, ainsi que, par un relargage progressif, une meilleure lubrification de l'épiderme, procurant à la fois un meilleur fonctionnement physiologique et un meilleur aspect au regard et au toucher.

### Exemple XI : Stabilisation du rapport CS/CNS

On traite une partie des échantillons de l'exemple précédent pour déterminer les teneurs en collagène, soluble et insoluble (CS et CNS).

A cet effet, après la première manipulation ayant abouti à l'extraction des lipides libres, le résidu est soumis à 5 reprises de 24 heures et à basse température, à un tampon calcique neutre (CTC = calcium tris citrate), puis les extraits sont réunis et dialysés. Il se produit alors un précipité contenant les protéines non solubles en l'absence de sels.

Par centrifugation, on obtient un culot et un surnageant. Le culot est resuspendu dans de l'eau distillée. Il contient le "collagène soluble cru". Quant au surnageant, il représente l'extrait CTC des protéines dites solubles.

Le résidu de l'extraction CTC est soumis à 5 reprises à une extraction par l'acide trichloracétique (TCA) à 2,7 % pendant des durées de 30 puis de 10 minutes, à la température de 90 °C. L'extrait TCA dialysé contient le collagène insoluble.

Le tableau ci-après expose les résultats obtenus, pour les peaux traitées avec la crème de l'exemple I ou avec son seul excipient, chacun en comparaison avec les témoins n'ayant reçu que du sérum physiologique. Les teneurs indiquées sont des taux moyens par sujet exprimés en milligrammes par gramme de poids sec, et l'on en a déduit le rapport CS/CNS :

| Collagène | soluble mg/g | insoluble mg/g | Rapport CS/CNS |
|---|---|---|---|
| Excipient | | | |
| Témoin | 34,03 | 28,07 | 1,21 |
| Excipient | 34,87 | 29,18 | 1,19 |

| Crème de l'invention | | | |
|---|---|---|---|
| Témoin | 34,97 | 28,68 | 1,24 |
| Crème | 38,41 | 24,43 | 1,58 |

Il apparaît ainsi qu'avec la crème classique, on ne constate pas d'effet significatif, alors que la crème selon l'invention fait augmenter le collagène soluble et diminuer le collagène insoluble. Cet effet entraîne une différence statistiquement très significative entre les rapports CS/CNS. On notera que l'évolution de ce rapport obtenue par l'application des compositions de l'invention va dans le sens correspondant à l'eutrophie du capital collagénique de la peau propre à la jeunesse.

### Exemple XII : Pouvoir protecteur des structures fibreuses

On sait qu'au cours du vieillissement, l'action des protéases fibrolytiques (collagénase et élastase) prend le pas sur l'activité réparatrice des cellules fibrocytaires et que ce phénomène est aggravé par les altérations de l'épithélium.

Les essais rapportés dans cet exemple ont été réalisés en double insu, sur des animaux vivants, pour le collagène et pour l'élastine. Ils démontrent que l'application in vivo des compositions cosmétiques au chromone-carboxylate-2 de diéthylamine augmente la capacité de résistance des fibres conjonctives de la peau aux agressions enzymatiques.

Trois groupes de 10 animaux (rats Wistar mâles) ont reçu chacun une crème différente : l'une était constituée par l'excipient seul de la crème selon l'exemple II et les deux autres par le même excipient auquel avait été incorporé le chromone-carboxylate de diéthylamine aux deux concentrations différentes des exemples II et III. Les expérimentateurs ignoraient que l'une des crèmes n'était que l'excipient.

Pour obtenir une bonne valeur statistique de ces tests, les animaux étaient leurs propres témoins comme dans les tests précédents. En effet les applications étaient faites sur les deux côtés rasés des animaux ; elles étaient quotidiennes et elles ont duré 21 jours. D'un côté, sur des zones bien délimitées, on appliquait le sérum physiologique, et de l'autre côté on appliquait la crème. Chaque onction était faite dans les mêmes conditions de quantité, de durée, et de surface d'application.

Après traitement, les peaux étaient prélevées et livrées aux experts. A l'exception des prélèvements destinés à constituer les témoins normaux, les fragments ont été exposés à des solutions, toujours identiques, de collagénase pour l'examen du collagène, ou d'élastase pour l'examen de l'élastine. Le taux de concentration des enzymes était choisi de façon à obtenir, dans les peaux non traitées par une crème de l'invention, une dégradation très importante, mais non totale.

Il est à noter que les peaux n'ont reçu du chromone-carboxylate que sur les sujets vivants, à l'aide des onctions de crèmes, et jamais sur les fragments de peau prélévés. Il est donc clair que les résultats présentés sont obtenus dans les conditions qui sont celles de l'utilisation en cosmétique, ce qui ne pourrait être soutenu dans le cas d'applications locales "in vitro" sur les prélèvements ou sur des compositions obtenues à partir de fibres conjonctives isolées de leur contexte tissulaire.

Le grossissement de G = 28 a permis de chiffrer sur une large plage, en utilisant la méthode de "morphométrie automatique" (analyse d'image par ordinateur), l'économie de fibres conjonctives procurées par l'application des crèmes des exemples II et III, comparativement aux peaux n'ayant reçu que l'excipient. Les résultats numériques sont exposés dans les tableaux ci-dessous, exprimés en pixels (point image) :

| Collagénase | Surface pixels | % de l'original |
|---|---|---|
| Témoins | 44345,0 | 100,00 |
| Excipient | 4535,8 | 10,22 |
| Crème Ex. II | 28647,1 | 64,46 |
| Crème Ex. III | 13957,1 | 31,47 |

| Elastase | | |
|---|---|---|
| Témoins | 2947,1 | 100,00 |
| Excipient | 955,9 | 32,40 |
| Crème Ex. II | 1571,8 | 53,33 |
| Crème Ex. III | 1364,2 | 46,29 |

On voit qu'à la suite de l'application des crèmes au chromone-carboxylate de diéthylamine II (5 %) et III (2,5 %), le collagène et l'élastine ont été protégés significativement alors que la crème excipient (sans chromone-carboxylate) n'a exercé aucune protection.

L'application sur la peau de sujets vivants de préparations cosmétiques comprenant le principe actif de l'invention entraîne donc une augmentation significative de la résistance des macromolécules fibreuses de la peau à l'action de la collagénase et de l'élastase, ce qui est d'un intérêt évident en matière de protection contre le vieillissement naturel ou accéléré par les agents exogènes.

## Revendications

1. Utilisation des sels d'acide chromone-carboxylique, dans la fabrication de nouveaux cosmétiques, efficaces en application locale sur la peau, pour lutter contre le vieillissement de celle-ci.

2. Composition cosmétique pour application locale sur l'épiderme cutané, caractérisé en ce qu'elle comporte comme constituant actif contre le vieillissement de la peau, le chromone-carboxylate-2 de sodium, potassium ou magnésium.

3. Composition selon la revendication 2, caractérisée en ce que ledit sel y est présent dans une proportion pondérale comprise entre 1 et 10 % du poids total de la composition, et notamment entre 2 et 8 %.

4. Composition selon l'une quelconque des revendications 2 à 3, caractérisée en ce qu'elle est sous forme d'un gel ou d'une lotion hydrique ou hydroalcoolique ou sous forme d'une crème ou d'un lait.

5. Composition selon l'une quelconque des revendications 2 à 4, sous la forme d'une émulsion huile dans eau, caractérisée en ce que ledit excipient comporte, en pourcentage en poids du poids total de la composition :
| | |
|---|---|
| Conservateurs | 0,1 à 0,5 |
| Emulsionnants non ioniques | 10 à 25 |
| Huiles végétales et synthétiques | 5 à 15 |
| Facteurs hydratants | 1 à 10 |

6. Procédé de traitement cosmétique de la peau, suivant lequel on applique localement sur l'épiderme cutané une composition cosmétique contenant un sel d'acide chromone-carboxylique en proportion efficace comme constituant actif contre le vieillissement de la peau.

## Claims

1. Use of salts of chromone carboxylic acid in the production of new cosmetics efficient when applied locally on skin to fight skin aging.

2. A cosmetic composition for local application on skin epiderm comprising as a constituent active against skin aging the chromone-2-carboxylate of sodium, potassium or magnesium.

3. A composition as claimed in claim 2 wherein said salt is present in a proportion comprised between 1 and 10 % of the total weight of the composition, and preferably between 2 to 8 %.

4. A composition as claimed in any of the claims from 2 to 3, in the form of a gel or a hydric or hydroalcoholic lotion, or in the form of a cream or milk.

5. A composition as claimed in any of the claims from 2 to 4, in the form of an oil in water emulsion, wherein said excipient comprises in percentage of the total weight of the composition :
| | |
|---|---|
| Preservatives | from 0,1 to 0,5 |
| Non ionic emulsifiers | from 10 to 25 |
| Vegetable and synthetic oils | from 5 to 15 |
| Moisturizing agents | from 1 to 10 |

6. A process for the cosmetic treatment of the skin wherein a cosmetic composition comprising a salt of chromone carboxylic acid in a proportion efficient to fight against skin aging, is applied locally on skin epiderm.

## Patentansprüche

1. Verwendung von Salzen der Chromon-carboxylsäure zur Herstellung neuer Kosmetika, die bei lokalen Auftragung auf die Haut deren Alterung bekämpfen.

2. Kosmetische Zusammensetzung zur lokalen Auftragung auf die Epidermis der Haut, dadurch gekennzeichnet, daß sie als aktiven Bestandteil gegen die Alterung der Haut die Natrium-, Kalium- oder Magnesiumsalze der Chromon-carboxylsäure-2 umfaßt.

3. Zusammensetzung nach Anspruch 2, dadurch gekennzeichnet, daß dieses Salz in einem Gewichtsanteil zwischen 1 und 10 % und insbesondere zwischen 2 und 8 % des Gesamtgewichts der Zusammensetzung vorhanden ist.

4. Zusammensetzung nach einem der Ansprüche 2 bis 3, dadurch gekennzeichnet, daß sie in Form eines Gels oder einer wäßrigen oder wäßrig-alkoholischen Flüssigkeit oder in Form einer Creme oder einer Milch vorliegt.

5. Zusammensetzung nach einem der Ansprüche 2 bis 4 in Form einer Öl-in-Wasser-Emulsion, dadurch gekennzeichnet, daß die Grundmasse in Gewichtsprozent des Gesamtgewichts der Zusammensetzung
| | |
|---|---|
| Konservierungsmittel | 0,1 bis 0,5 |
| nichtionische Emulgatoren | 10 bis 25 |
| pflanzliche und synthetische Öle | 5 bis 15 |
| Feuchthaltemittel | 1 bis 10 |
umfaßt.

6. Verfahren zur kosmetischen Behandlung der Haut, bei dem lokal auf die Epidermis der Haut eine kosmetische Zusammensetzung aufgetragen wird, die ein Salz der Chromon-carbonsäure in wirksamem Anteil als aktiven Bestandteil gegen die Alterung der Haut enthält.
